# EUROPEAN PATENT APPLICATION

(11) **EP 3 906 884 A1**
(43) Date of publication of application: **10.11.2021**
(21) Application number: 20172786.4
(22) Date of filing: 04.05.2020
(51) Int. Cl.: A61B 90/30, A61B 90/35, F21V 21/26, A61B 90/50, F21V 21/28, A61G 15/00, A61C 19/00, A61B 90/00

(54) **OPERATING LAMP**

(71) Applicant: F.A.R.O. FABBRICA APPARECCHIATURE RAZIONALI ODONTOIATRICHE S.p.A., 20876 Ornago (MB) (IT)
(72) Inventor: BACCO, Emiliano, I-20882 Bellusco (MB) (IT); FIORELLO, Cristian, I-20871 Vimercate (MB) (IT); FAVONIO, Angelo, I-20871 Vimercate (MB) (IT)
(74) Representative: Porta & Consulenti Associati S.p.A.

(57) **Abstract**

An operating lamp (1) comprises a light head (10); a first body (30); a second body (50) rotatably connected to said first body (30) about a main axis of rotation (R); an intermediate body (40) arranged between said first and second body (30, 50) and rotatable about said main axis of rotation (R); a first limitation mechanism (60) comprising a first element and a second element; and a second limitation mechanism (70) comprising a first element (71) and a second element (72); wherein the first element (61) and the second element (62) of the first limitation mechanism (60) cooperate to limit a rotation of the first body (30) with respect to the intermediate body (40) about the main axis of rotation (R) to a value less than 359°; and wherein the first element (71) and the second element (72) of the second limitation mechanism (70) cooperate to limit a rotation of the intermediate body (40) with respect to the second body (50) about the main axis of rotation (R) to a value less than 359°.

## Description

The present invention relates to an operating lamp.

Some technical sectors have the peculiarity of envisaging that the specialized operator performs their activity focusing their attention and intervention in a predetermined operating field, the latter being understood as the physical sector or space in which the operator operates.

An example of technical sectors of this nature is dentistry, in which the operating field substantially coincides with the patient's mouth.

Other examples of this type of technical sector are surgery, in which the surgeon operates in an operating field (also in this case strongly lit) in which the patient lies, and veterinary medicine which has similar lighting requirements.

A further example is represented by jewellery making, where the goldsmith operates with his/her tools in a strongly lit operating field to enable the perfect lighting of the jewellery that he/she is making.

An operating lamp means a lamp used to illuminate the operating field with very intense light.

Other peculiarities of operating lamps are the marked directionality of the beam of light emitted and the possibility to orient it according to requirements.

A typical example of an operating lamp, to which explicit reference will be made, is the dental lamp.

Dental lamps are able to illuminate the patient's oral cavity with light intensity that typically exceeds 20000 lux and can reach up to 70000 lux and which is focused in a beam which, from a distance of about 70 centimetres, illuminates the patient's oral cavity.

The present invention further relates to an operating unit, e.g. a dentistry or veterinary unit which comprises a support for the patient. The operating lamp may be integrated into the unit or it may be independent from the unit and fixed to a wall or to the ceiling of the room.

In the dentistry field the support consists of a reclining chair for accommodating the patient and allowing the dentist to operate on him/her. The activity of the operators, such as dentists and dental hygienists, takes place around the chair. The chair can be configured to modify the position of the patient according to specific requirements.

The dental unit further comprises other devices such as, for example, a case on which the various dental tools are mounted, interface commands (typically a keyboard and a pedal) for regulating the chair, the operating tools, trays for the tools and materials used by the dentist, saliva ejector, cup holder, dental spittoon. The dental unit is connected to a medical compressor able to dispense clean dry air and a surgical aspirator able to generate suction for removing water, saliva and blood from the patient's mouth.

The operating lamp must be able to be easily moved in order to adapt the position and orientation of the beam of light to the type of operations and the position of the operators, so that the latter do not block the light. For example, in the dental field, the position of the dental lamp must be able to be adapted in order to obtain optimal lighting of the oral cavity. The operating lamp must also be able to be moved to prevent it being able to interfere with the operations or obstruct the operator.

For that purpose, the operating lamps are mounted on an articulated arm that enables the operator to modify the spatial position of the lamp. The lamp is also provided with one or more articulations that enable the beam of light emitted by the lamp to be directed with respect to the articulated arm. The articulated arm can be fixed to the unit (in the event in which the lamp is part of the unit) or to a wall or ceiling (in the case in which the lamp is independent from the unit).

Each articulation can be made of a pin rotatably inserted in a cavity to enable the reciprocal rotation of the lamp with respect to the articulated arm about an axis of rotation. The supply cables of the lamp and/or control cables of the lamp pass through the pin and the cavity reaching the lamp.

Each articulation is typically provided with a mechanical end stop which aims to limit the maximum angle of rotation of the lamp with respect to the arm. Typically, the mechanical end stop is made of an arched guide obtained on the lamp or on the articulated arm engaged slidably by a key obtained on the arm or on the lamp.

The angular range of the arched guide defines the maximum angle of rotation of the lamp with respect to the arm.

The mechanical end stop is necessary for preventing the power supply and/or control cables of the lamp from getting twisted on themselves until the inevitable damage of the cables or of the connection cables with the lamp.

In fact, should the operator make the lamp perform a plurality of rotations of the lamp with respect to the arm always in the same angular direction, the power supply and/or control cables of the lamp would continue to twist onto themselves until an inevitable breakage occurs.

The mechanical end stop stops the rotation of the lamp with respect to the arm in both angular directions.

The Applicant has verified that the mechanical end stops briefly described above limit the angular range of the articulation to an extension less than a full circle.

The Applicant has noted that this precludes some configurations of the lamp with respect to the articulated arm.

The Applicant observes that, in relation to the dental area, with the progress of dentistry, the tendency to arrange the patient in different positions from the semi-lying down or lying down positions has grown. For example, it is consolidated practice to arrange the patient in the erect sitting position to evaluate aspects that can be influenced by the force of gravity, such as for example chewing.

In the Applicant's experience, if the patient is positioned in some positions the mechanical end stops applied to the articulations constitute an obstruction to the arrangement of the lamp in order to direct the beam of light towards the patient's oral cavity and so as not to obstruct the operator's operations.

For example, the Applicant has noted that in some configurations of the articulated arm the articulation is proximal to the end stop. Therefore, apparently small adjustments of the position or orientation of the beam of light can require the repositioning of the lamp and the entire articulated arm. This repositioning is not very practical to perform and not always possible, as it may occur that the new position of the articulated arm obstructs the movements of the operators or of other devices mounted on the dental unit such as monitor arms, microscope or the like.

The Applicant has perceived that, if the angular range of the lamp increases with respect to the articulated arm beyond a full circle and without losing the possibility to arrange a mechanical end stop, the lighting of the operating field, the work conditions of the operators and the performance levels provided thereby could be improved, without compromising the integrity and functionality of the lamp.

The above description in relation to the dental field also applies to operating lamps and operating units used in sectors (such as for example the veterinary and jewellery making sectors) in which it is necessary for the operating lamp to be easily movable in order to adapt the position and direction of the beam of light to the specific operating field and the specific position of the operator.

Therefore, in a first aspect thereof, the present invention relates to an operating lamp comprising:
- a light head;
- a first body connected to said light head;
- a second body rotatably connected to said first body about a main axis of rotation;
- an intermediate body arranged between said first and second body and rotatable about said main axis of rotation with respect to said first body and with respect to said second body;
- a first limitation mechanism comprising:
   a first element arranged on the intermediate body and a second element arranged on the first body, or
   a first element arranged on the first body and a second element arranged on the intermediate body; and
- a second limitation mechanism comprising:
   a first element arranged on the intermediate body and a second element arranged on the second body, or
   a first element arranged on the second body and a second element arranged on the intermediate body;
wherein the first element and the second element of the first limitation mechanism cooperate to limit a rotation of the first body with respect to the intermediate body about the main axis of rotation to a value less than 359°; and
wherein the first element and the second element of the second limitation mechanism cooperate to limit a rotation of the intermediate body with respect to the second body about the main axis of rotation to a value less than 359°.

The light head projects a beam of light which enables the operator to illuminate the operating field, e.g. the patient's oral cavity in the case of use in the dental sector.

The first and second body enable the beam of light to be directed through the rotatable connection between them. In particular, the first body can be rigidly connected to the light head or rotatably constrained to the light head through one or more articulations. The second body can be rigidly connected to a part of a plurality of bodies connected by articulations that form an articulated arm, can be an integral part of the articulated arm or can be rotatably constrained to a part of the plurality of bodies connected to one another.

The intermediate body is arranged between the first body and the second body and can rotate both with respect to the first body and with respect to the second body.

The first and the second limitation mechanism limit the maximum rotation respectively of the first body with respect to the intermediate body and of the second body with respect to the intermediate body. The maximum rotation between the first body and the second body is equal to the sum of the respective maximum rotations with respect to the intermediate body. Advantageously, this feature enables a greater rotation to be obtained with respect to the one obtainable with a traditional mechanical end stop but still less than 718°, a value that enables the integrity of any electrical cables that cross the first and the second body for supplying and regulating the light source of the light head to be protected.

In a second aspect thereof, the present invention relates to an operating lamp assembly comprising:
- an articulated arm;
- an operating lamp according to the first aspect of the invention coupled to the articulated arm.

In a third aspect thereof, the present invention relates to an operating unit comprising:
- a chair;
- an articulated arm coupled to the chair;
- an operating lamp according to the first aspect of the invention coupled to the articulated arm.

In the present description and subsequent claims, the term "body" means a single mechanical element made as one part or as a plurality of single parts integral with one another.

The first and the second body are rotatable about a main axis of rotation. Such main axis of rotation is the main reference axis for the elements that are part of the present invention; the direction and other indications will be referred with respect thereto, such as "axial", "radial", "circumferential", "diametrical", unless a different reference axis is explicitly specified. The indications "internal", "internally" and "external", "externally" refer to the position of the elements with respect to the axis of rotation and indicate respectively a closer or further position to/from the main axis of rotation. With respect to the main axis of rotation, two opposite rotation directions are defined, in particular a first angular direction and a second angular direction.

In at least one of the aforesaid aspects, the present invention can present at least one of the preferred characteristics described below considered singularly or in combination.

Preferably, the sum of the maximum rotation permitted for the first body with respect to the intermediate body by the first limitation mechanism and the maximum rotation permitted for the intermediate body with respect to the second body by the second limitation mechanism is greater than 361°.

In other words, the first and the second limitation mechanism are configured to enable a rotation of the first body with respect to the second body about the main axis of rotation greater than 361°. This value enables there to be no blind spots and the light head to be oriented in directions proximal to the end stops both through a rotation in the first angular direction and through a rotation in the second angular direction.

Preferably, the first element of the first limitation mechanism comprises a first end stop configured to cooperate with the second element of the first limitation mechanism for stopping the rotation of the first body with respect to the intermediate body along a first angular direction.

Preferably, the first element of the first limitation mechanism comprises a second end stop configured to cooperate with the second element of the first limitation mechanism for stopping the rotation of the first body with respect to the intermediate body along a second angular direction opposite the first angular direction.

Preferably, the first element of the second limitation mechanism comprises a first end stop configured to cooperate with the second element of the second limitation mechanism for stopping the rotation of the intermediate body with respect to the second body along a first angular direction.

Preferably, the first element of the second limitation mechanism comprises a second end stop configured to cooperate with the second element of the second limitation mechanism for stopping the rotation of the intermediate body with respect to the second body along a second angular direction opposite the first angular direction. Preferably, the second element of the first limitation mechanism comprises a slider configured to cooperate with the first element of the first limitation mechanism to stop the rotation of the first body with respect to the intermediate body.

Preferably, the second element of the second limitation mechanism comprises a slider configured to cooperate with the first element of the second limitation mechanism to stop the rotation of the intermediate body with respect to the second body.

The slider of the first limitation mechanism is movable along a respective circular trajectory during a relative rotation between the first body and the intermediate body about the main axis of rotation. The respective end stops are arranged along the trajectory of the slider so as to intercept the slider and stop such rotation when it reaches the maximum envisaged value. Likewise, the slider of the second limitation mechanism is movable along a respective circular trajectory during a relative rotation between the second body and the intermediate body about the main axis of rotation and the respective end stops are arranged along its trajectory so as to intercept it and stop such rotation so as to limit it when it reaches the maximum envisaged value.

Preferably, the first element of the first limitation mechanism comprises a guide track between the respective first end stop and the second end stop, the slider of the first limitation mechanism being configured to slide in said guide track during a rotation of the first body with respect to the intermediate body.

Preferably, the first element of the second limitation mechanism comprises a guide track between the respective first end stop and the second end stop, the slider of the second limitation mechanism being configured to slide in said guide track during a rotation of the intermediate body with respect to the second body.

The end stop tracks enable the sliders to move between two end stops during the rotation of the first body and of the second body with respect to the intermediate body within the predefined limits.

Preferably, the first body and the second body are rotatably connected by means of a pin that extends along said main axis of rotation, wherein the intermediate body is crossed by said pin.

The pin connects the first body to the second body, keeps in position the intermediate body and determines the fulcrum of the reciprocal rotations of the first body, the second body and the intermediate body.

In one embodiment the pin is integral with the first body.

Alternatively, the pin is integral with the second body.

Preferably, the intermediate body has a radially internal surface comprising a first portion conformed to slide on said pin for rotatably coupling said intermediate body to said pin.

The intermediate body can rotate about the pin in the angular limits defined by the first and the second limitation mechanism. The first portion of the radially internal surface slides around the pin to define the kinematics of such rotation.

Preferably, the first portion of the radially internal surface of the intermediate body has a circumferential extension such as to at least partially embrace the pin for rotatably constraining the intermediate body to the pin.

Preferably, the first portion of the radially internal angular surface of the intermediate body has an angular extension about the main axis of rotation that defines an angle with respect to the main axis of rotation greater than 180°, preferably comprised between 210° and 330°, for example equal to about 300°.

Preferably, the radially internal surface of the intermediate body has a second portion circumferentially extending from the first and the second end stop of one from between the first and the second limitation mechanism. The second portion of the radially internal surface is placed at a greater radial distance from the main axis of rotation than the radial distance of the first portion of the radially internal surface from the main axis of rotation.

Preferably, the guide track of said one from between the first and the second limitation mechanism is radially delimited by the second portion of the radially internal surface of the intermediate body and circumferentially delimited by the first and the second end stop of said one from between the first and the second limitation mechanism.

Preferably, the second portion of the radially internal surface directly faces the pin and the guide track of said one from between the first and the second limitation mechanism is delimited in the radially internal direction by the pin.

In other words, in an embodiment of the operating lamp, the intermediate body has a hole crossed by the pin and the guide track is part of the hole. The hole performs the dual function of fitting the intermediate body onto the pin and limiting the rotation of the intermediate body in cooperation with the respective slider. In particular, the hole represents the first portion of the radially internal surface that is shaped so as to embrace the pin and slide thereon, enabling the rotation of the intermediate body with respect to the pin and at the same time keeping the intermediate body constrained thereto. Furthermore, the hole has the first and second end stop, which intercept the slider for stopping the rotation of the intermediate body, defined by a connection portion between the first and the second portion of the radially internal surface. This simplifies the structure of the intermediate body and of the lamp as a whole.

Preferably, the second portion of the radially internal surface has an angular extension around the main axis of rotation that defines an angle with respect to the main angle of rotation less than 300°, more preferably less than 180°, preferably comprised between 30° and 120°, for example equal to about 90°.

Preferably, the angular extension of the first portion of the radially internal surface added to the angular extension of the second portion of the radially internal surface is equal to about 360°.

The angular extension of the second portion of the radially internal surface defines a respective extension guide track sufficient to enable a total rotation of the first body with respect to the second body greater than a full circle.

Preferably, the first portion of the radially internal surface is circumferentially extended from the first to the second end stop and is complementary, with respect to a full circle, to the second portion of the radially internal surface.

Preferably, the first portion and the second portion of the radially internal surface have respective constant radii with respect to the main axis of rotation.

In other words, in one embodiment, the radially internal edge of the hole is subdivided into the first portion and the second portion of the radially internal surface and the difference between the radius of the second portion of the radially internal surface and the radius of the first portion of the radially internal surface is substantially equal to the radial extension of the two end stops.

In one embodiment, the pin is integral with the first body and the slider of the first limitation mechanism is integral and adjacent to the pin and engages the guide track of the first limitation mechanism. In this embodiment the second portion of the radially internal surface defines the guide track of the first limitation mechanism.

Alternatively, the pin is integral with the second body and the slider of the second limitation mechanism is integral and adjacent to the pin and engages the guide track of the second limitation mechanism. In this embodiment the second portion of the radially internal surface defines the guide track of the second limitation mechanism.

The pin and the slider are integral to one another. The slider may be adjacent to the pin and fixed thereto and project radially for limiting the rotation of the intermediate body. This feature simplifies the structure of the intermediate body and of the limitation mechanism.

Preferably, the first body comprises an abutment portion arranged in contact abutment against the intermediate body.

Preferably, the second body comprises an abutment portion arranged in contact abutment against the intermediate body.

In one embodiment, the guide track of the first limitation mechanism is defined by a groove obtained on the abutment portion of the first body and circumferentially extended around the main axis of rotation from the first end stop to the second end stop of the first limitation mechanism. Preferably, the slider of the first limitation mechanism is connected to the intermediate body and extends axially into the groove.

In one embodiment, the guide track of the second limitation mechanism is defined by a groove obtained on the abutment portion of the second body and circumferentially extended around the main axis of rotation from the first end stop to the second end stop of the second limitation mechanism. Preferably, the slider of the first limitation mechanism is connected to the intermediate body and extends axially into the groove.

Preferably, the groove has a circumferential extension around the main axis of rotation that defines an angle with respect to the main axis of rotation greater than 180°, preferably greater than 270°, even more preferably greater than 300°, even more preferably greater than 330°, for example comprised between 350° and 359°.

The configuration of the abutment portion and the extension of the groove enable the rotatable coupling of the first (or of the second) body with the intermediate body and enable a sufficient rotation with respect to the intermediate body such as to enable a total rotation between the first and the second body greater than a full circle.

Preferably, the intermediate body comprises a washer having a first flat surface arranged in contact support against said first body and a second flat surface opposite the first flat surface and arranged in contact support against said second body.

Preferably, the washer is crossed by the pin.

Preferably, the washer has the hole crossed by the pin and the hole has a lobe defining the second portion of the radially internal surface and the first and second end stop of the first and second limitation mechanism.

Preferably, the washer has a thickness less than 3 mm and even more preferably less than 1.5 mm.

In one embodiment, the washer has the first and the second flat surface coated with an anti-friction material (e.g. Teflon) or treated superficially for reducing the friction thereof (e.g. through PVD (Physical Vapor Deposition) or CVD (Chemical Vapor Deposition)).

This feature reduces the rotation friction between the two bodies during the reciprocal rotations and can make it possible to prevent the use of lubricant oils or greases.

Preferably, the first body has a first cavity and the second body has a second cavity in communication with the first cavity.

Preferably, the pin is hollow and places in communication the first cavity and the second cavity.

Preferably, a plurality of electric conductors extends from said first body and said second body in the first and second cavity.

Preferably, the pin is crossed by the plurality of electric conductors.

The conductors are housed in the cavities and are therefore protected by the first and second body and hidden from view. The hollow structure of the pin and the limitation on the rotation determined by the first and the second limitation mechanism enables the integrity of the conductors to be protected.

Preferably, the light head has a main light axis and the rotation between the first body and the second body modifies the direction of the main light axis.

Preferably, the light head is crossed by the main axis of rotation.

In this way, by rotating the first body with respect to the second body the main light axis is oriented without the light head performing any significant spatial displacements.

Preferably, the second body is connected to a translation body, the translation body housing an articulated parallelogram configured to make the light head translate along the main axis of rotation and to maintain a constant inclination of the main light axis during such translation.

Preferably, the operating lamp is a dental lamp.

Preferably, the operating unit is a dental unit.

Further characteristics and advantages of the present description will become clearer from the following detailed description of the preferred embodiments thereof, with reference to the appended drawings and provided by way of indicative and non-limiting example. In such drawings, in which the embodiments are not scale representations:
- Figure 1 shows a perspective view of an operating lamp assembly and an operating lamp according to the present invention;
- Figure 2 shows an exploded view of a detail of an operating lamp according to the present invention;
- Figure 3 shows a perspective view of a detail of an operating lamp according to the present invention;
- Figure 4 shows a perspective view of a detail of an operating lamp according to the present invention;
- Figure 5 shows a perspective view of a detail of an operating lamp according to the present invention;
- Figure 6 shows a sectional view of a detail of an operating lamp according to the present invention;
- Figure 7 shows a sectional view of a detail of an operating lamp according to the present invention;
- Figure 8 shows a sectional view of an operating unit according to the present invention.

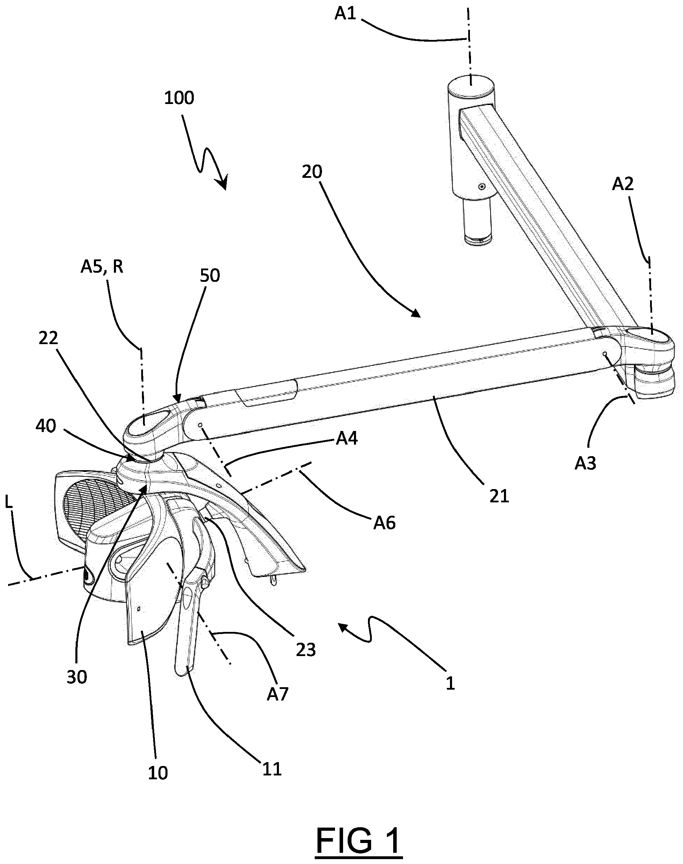

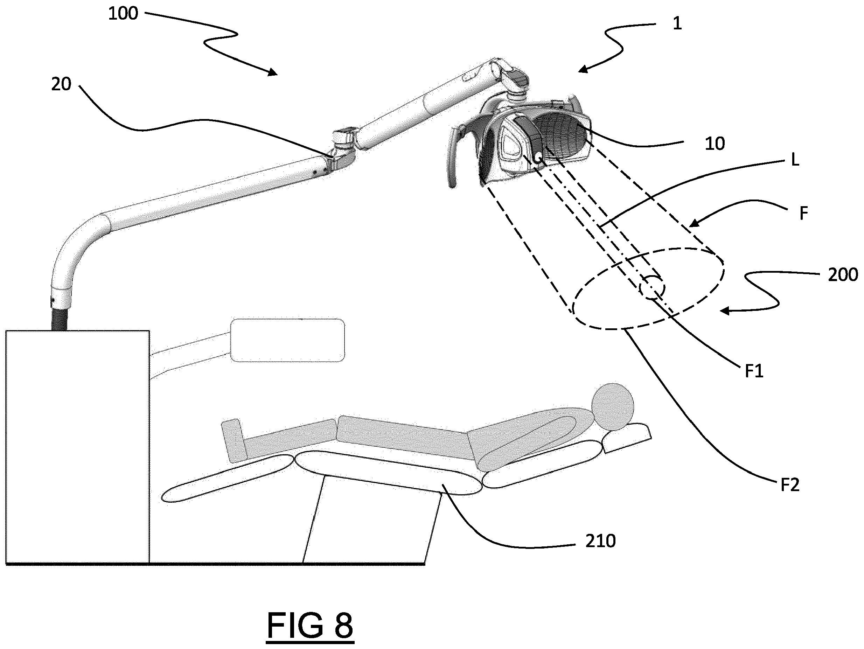

An operating lamp according to the present invention is illustrated in Figures 1 and 8 where it is generally indicated with reference number 1. The operating lamp 1 is preferably a dental lamp.

The operating lamp 1 comprises a light head 10. The light head 10 is configured to emit a beam of light F so as to enable an operator to illuminate an operating field. In the event of use in the dental field, the operating field is the patient's oral cavity and the light head 10 is configured to illuminate it according to the methods envisaged by legislation and standards in dentistry.

The beam of light F has a main light axis L, which is essentially defined by the projection direction of the maximum light intensity emitted by the light head 10.

In the event of use in the dental field, as illustrated in Figure 8, the beam of light emitted by the light head 10 has a region with a higher intensity F1 and a region with a lower intensity F2. The region with the highest intensity F1 is concentrated around the main light axis L and configured, subject to suitable positioning of the light head 10, to illuminate the patient's oral cavity with substantially uniform intensity comprised between 8000 lux and 70000 lux. The region with the lowest intensity F2 is distributed around the region with the highest intensity F1, further from the main light axis L, and configured to illuminate the area overlooking the patient's oral cavity with a lower intensity than that of the region with greater intensity F1.

In the embodiment illustrated, the light head 10 comprises a plurality of reflecting bodies and a pair of light sources (preferably LED) that project light onto them. The plurality of reflecting bodies is arranged around the main light source L and configured to reflect the light emitted by the light sources so as to project the beam of light along the main light axis L with the intensity distribution described above.

The operating lamp 1 comprises two handles 12 connected to the light head 10 and projecting in opposite directions so as to be easily grasped by the operator.

An articulated arm 20, illustrated in Figures 1 and 8, is connected to the operating lamp 1 to support it and enable the movement thereof. The articulated arm 20 houses the electric cables (not illustrated) that take the supply and control current to the light head 10. The articulated arm 20 comprises a plurality of bodies connected to one another by rotatable articulations having respective axes of rotation A1, A2, A3, A4. The reciprocal rotation of the bodies of the articulated arm 20 modifies the spatial position of the light head 10 and the orientation of the main beam of light.

In particular, the articulations between bodies are configured to keep the light head 10 firmly in a stationary position in the absence of significant forces acting thereon, whatever its position and orientation. The resistance to rotation of the various articulations is calibrated to prevent accidental displacements of the light head 10 and to enable the operator to change the position thereof by applying a manual force thereto by means of the hand grip 11. Following the application of such force, the bodies of the articulated arm 20 are oriented so as to accommodate the configuration of the light head 10 desired by the operator.

The articulated arm 20 comprises two distal articulations having the first axis of rotation A1 and the second axis of rotation A2, respectively, parallel to one another. Such distal articulations enable the movement of the lamp in a work plane perpendicular to the first A1 and second axis of rotation A2. The work plane is sufficiently extensive to enable the movement of the light head 10 in the entire operating area.

The articulated arm 20 further comprises a regulation body 21 having one end connected to the distal articulations. The regulation body 21 has a third axis of rotation A3 and a fourth axis of rotation A4, both perpendicular to the first A1 and second axis of rotation A2, and comprises an articulated parallelogram (or pantograph) configured to enable a movement of the light head 10 in the perpendicular direction to the work plane (i.e. parallel to the first A1 and second axis of rotation A2).

Figures 1 and 8 show an operating lamp assembly 100, comprising the operating lamp 1 and the articulated arm 20.

The operating lamp 1 comprises a first body 30 and a second body 50 rotatably connected to one another to define a main axis of rotation R.

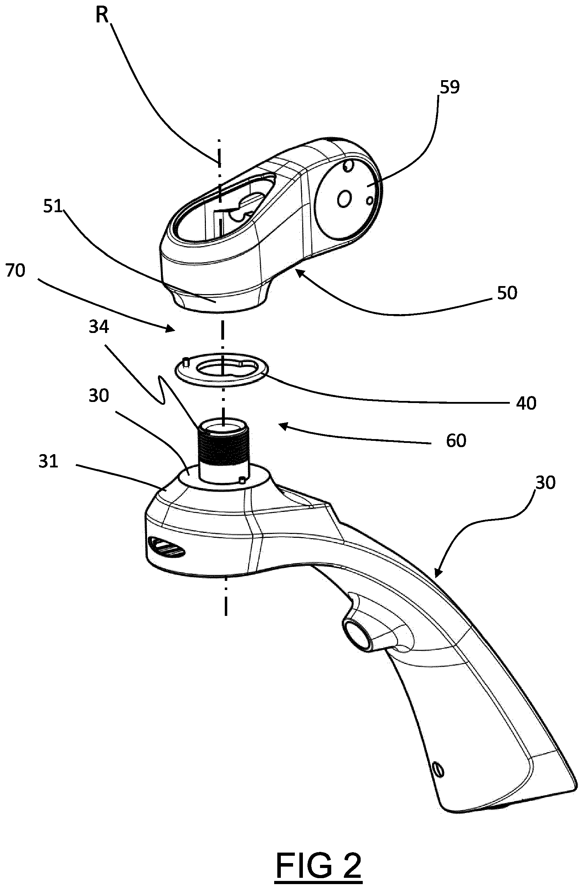

The first 30 and the second body 50 define, along with an intermediate body 40, axially interposed between the first 30 and the second body 50, a first proximal articulation 22 which enables the first body 30 and the second body 50 to rotate about a respective axis of rotation A5. The first proximal articulation is better illustrated in the exploded view in Figure 2.

It is to be noted that the axis of rotation A5 of the first proximal articulation 22 coincides with the main axis of rotation R.

The first body 30 is connected to the light head 10 and the second body 50 is connected to the articulated arm 20.

The first body 30 is, in the embodiment illustrated in the appended figures, connected to the light head 10 through a second proximal articulation 23, which enables the light head 10 to rotate with respect to the first body 30 about a respective axis of rotation A6, inclined with respect to the axis of rotation A5 of the first proximal articulation 22.

The first body 30 is further connected to the light head 10 through a third proximal articulation (not illustrated) having an axis of rotation A7 substantially perpendicular to the main light axis L. The axis of rotation A7 of the third proximal articulation is further perpendicular to the axis of rotation A6 of the second proximal articulation 23.

The second body 50 is, in the embodiment illustrated in the appended figures, connected to the regulation body 21 through the fourth axis of rotation A4.

The articulated parallelogram is configured to keep the axis of rotation R of the first proximal articulation 22 oriented parallel to the axes of rotation A1 and A2 of the distal articulations. The first proximal articulation 22 enables a rotation of the light head 10 in the work plane in order to modify the orientation of the main light axis L.

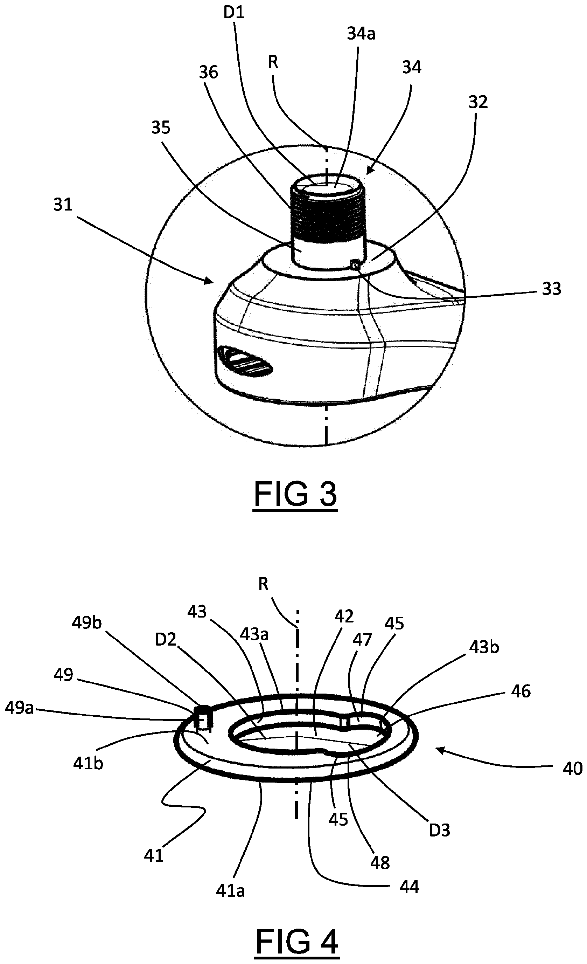

The first body 30 has an abutment portion 31, illustrated in Figure 3, having a flat and substantially smooth abutment surface 32.

The flat abutment surface 32 is substantially perpendicular to the main axis of rotation R.

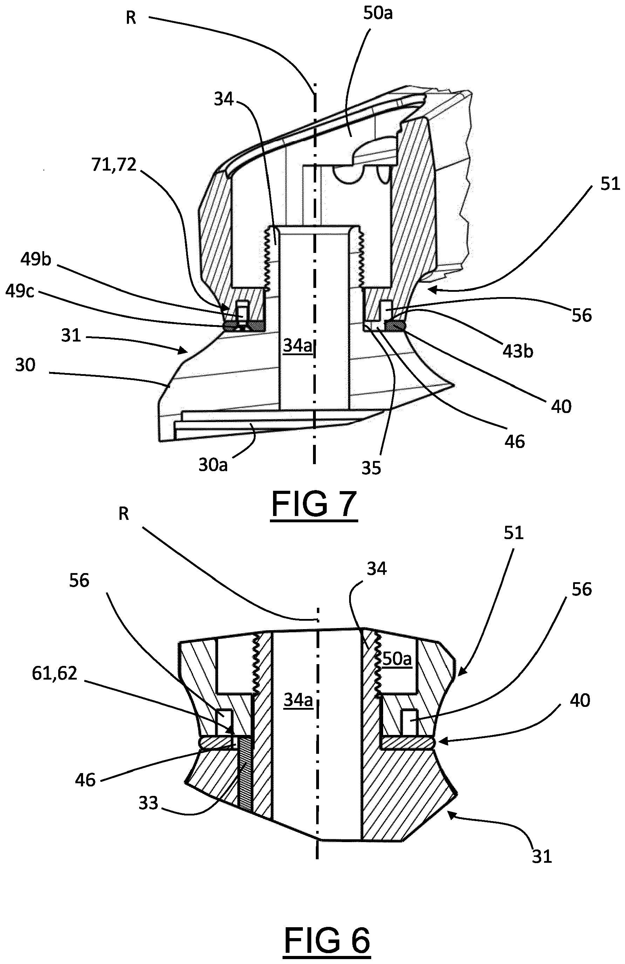

A slider 33 projects axially in relief with respect to the abutment surface 32. In the embodiment illustrated the slider 33 is a cylindrical insert partially inserted into the abutment portion 31 of the first body 30. Furthermore, the first body 30 has an internal cavity 30a illustrated in Figure 7.

A pin 34 projects axially from the abutment surface 32 perpendicular to the latter. The pin 34 has an external cylindrical surface 35 having a threaded portion 36. In the embodiment illustrated the pin 34 and the slider 33 are adjacent and integral. Furthermore, the pin 34 has an internal cavity 34a in communication with the internal cavity 30a of the first body 30.

The intermediate body 40, illustrated in Figure 4, is adjacent to the first body 30 and comprises a washer 41 having a thickness of about 2.5 mm.

The washer 41 has a first flat surface 41a, arranged in contact support against the abutment surface 32 of the first body 30, a second flat surface 41b opposite the first flat surface 41a and a hole 42 which extends from the first flat surface 41a to the second flat surface 41b. The hole 42 is crossed by the pin 34.

At the hole 42, the washer 41 has a radially internal surface 43 having a first portion 43a and a second portion 43b. The first portion 43a has the shape of an arc of a circle. The first portion 43a of the radially internal surface 43 has an angular extension of about 300°. The first portion 43a extends around the pin 34 so as to embrace it partially for rotatably constraining the washer 41 thereto. The difference between the outer radius D1 of the pin 34 and the radial distance D2 of the first portion 43a of the radially internal surface 43 from the main axis of rotation R is less than 0.5 mm so as to guarantee a rotatable coupling with minimum play between the washer 41 and the pin 34.

The intermediate body 40 rotates with respect to the pin 34 and to the first body 30 around the main axis of rotation R. During such rotation the first flat surface 41a of the washer 41 slides against the abutment surface 32 of the first body 30.

The second portion 43a of the radially internal surface 43 is shaped like an arc of a circle and has a radial distance D3 from the main axis of rotation R that is greater than the radial distance D2 of the first portion 43a of the radially internal surface 43 from the main axis of rotation R. In the embodiment illustrated, the second portion 43b of the radially internal surface 43 directly faces the pin 34. The second portion 43b of the radially internal surface 43 has an angular extension of about 60°. The first portion 43a and the second portion 43b of the radially internal surface 43 are connected by radially extending surfaces 45.

The washer 41 has a substantially circle shaped radially external surface 44.

The second portion 43b of the radially internal surface 43 and the radially extending surfaces 45 delimit a guide track 46 extending in an arc around the main axis of rotation R.

The slider 33 of the first body 30 engages the guide track 46 of the intermediate body 40 and moves within it during the rotation between the intermediate body 40 and the first body 30 with a circular trajectory with respect to the main axis of rotation R.

The radially extending surfaces 45 define a respective first end stop 47 and a respective second end stop 48 for the slider 33. The contact between the slider 33 and the first end stop 47 stops the reciprocal rotation between the first body 30 and the intermediate body 40 along a first angular direction. The contact between the slider 33 and the second end stop 47 stops the reciprocal rotation between the first body 30 and the intermediate body 40 along a second angular direction. In this way, the reciprocal rotation between the first body 30 and the intermediate body 40 is limited to the angular extension of the second portion 43b of the radially internal surface 43.

The slider 33 of the first body 30 and the guide track 46, the first end stop 47 and the second end stop 48 of the intermediate body 40 form part of a first limitation mechanism 60 configured to limit the reciprocal rotation between the first body 30 and the intermediate body 40. In particular, the slider 33 forms part of a first element 61 of the first limitation mechanism 60 and the guide track 46, the first end stop 47 and the second end stop 48 form part of a second element 62 of the first limitation mechanism 60 cooperating with the first element 61. The first limitation mechanism 60 is illustrated in detail in Figure 7 which shows a section along a plane containing the main axis of rotation R and the slider 33.

A further slider 49 is fixed to the washer 41 and projects from the second flat surface 41b in the parallel direction to the main axis of rotation R. In particular, the slider 49 comprises a bolt 49a having a stem 49b and a head 49c. Contextually, the washer 41 has a secondary threaded hole (not illustrated) having a housing for the head of the bolt 49a. The bolt 49a is screwed into the secondary hole so that the head is fully housed in the housing and the stem 49b projects from the second flat surface 41b.

The second body 50 is connected to the first body 30 through the pin 34 and can rotate with respect thereto about the main axis of rotation R. In particular, the second body 50 has a hole 50a that houses the pin 34. A threaded nut (not illustrated) can be screwed onto the threaded portion 36 of the pin 34 for clamping the second body 50 towards the first body 30.

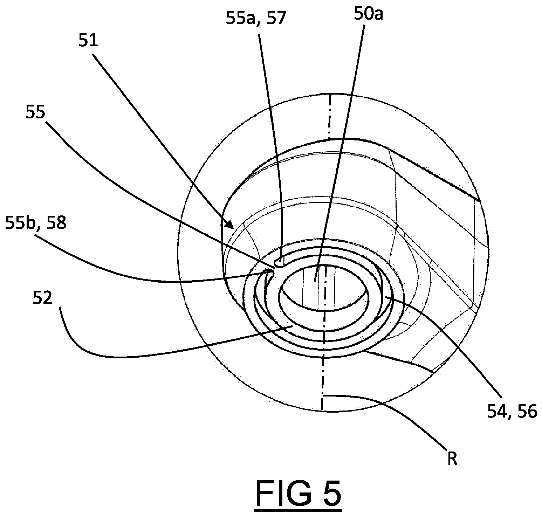

The second body 50 comprises an abutment portion 51, illustrated in Figure 5, having a flat abutment surface 52 in contact support against the second flat surface of the intermediate body 40. The flat abutment surface 52 is substantially perpendicular to the main axis of rotation R and parallel to the flat abutment surface 32 of the first body 30.

The second body 50 can rotate with respect to the intermediate body 40 about the main axis of rotation R independently with respect to the rotation between the intermediate body 40 and the first body 30. During such rotation the abutment surface 52 of the second body 50 slides against the second flat surface 41b of the intermediate body 40.

The abutment portion 51 of the second body 50 has a groove 54 extending circumferentially around the main axis of rotation R. The groove 54 has a depth dimension measured parallel to the main axis of rotation R of about 5 mm. The abutment portion 51 of the second body 50 has a partition 55 inserted into the groove 54. The angular extension of the groove around the main axis of rotation R is preferably comprised between 350° and 359° according to the thickness of the partition 54.

The groove 54 delimits a guide track 56 extending in an arc around the main axis of rotation R.

The further slider 49 projecting from the intermediate body 40 engages the guide track 56 of the second body 50 and moves within it during the rotation between the intermediate body 40 and the second body 50 with a circular trajectory with respect to the main axis of rotation R.

The partition 55 defines a first end stop 57 at a first wall 55a thereof and a second end stop 58 at a second wall 55b thereof. The contact between the further slider 49 and the first end stop 57 stops the reciprocal rotation between the second body 50 and the intermediate body 40 along a first angular direction. The contact between the further slider 49 and the second end stop 58 stops the reciprocal rotation between the second body 50 and the intermediate body 40 along the second angular direction. In this way, the reciprocal rotation between the second body 50 and the intermediate body 40 is limited to the angular extension of the groove 54 around the main axis of rotation R.

The further slider 49 of the intermediate body 40 and the guide track 56, the first end stop 57 and the second end stop 58 of the second body 50 form part of a second limitation mechanism 70 configured to limit the reciprocal rotation between the second body 50 and the intermediate body 40. In particular, the further slider 49 forms part of a first element 71 of the second limitation mechanism 70 and the guide track 56, the first end stop 57 and the second end stop 58 form part of a second element 72 of the second limitation mechanism 70 cooperating with the first element 71. The second limitation mechanism 70 is illustrated in detail in Figure 7 which shows a section along a different plane from the section plane of Figure 6 and containing the main axis of rotation R and the further slider 49.

The second body 50 comprises an end portion 59, integral with the abutment portion 51 thereof and connected to the regulation body 21. The end portion 59 is rotatable with respect to the regulation body 21 around the fourth axis of rotation A4. The articulated parallelogram of the regulation body 21 acts on the end portion 59 of the second body 50.

The second body 50 has an internal cavity 50a in communication with the internal cavity 30a of the first body 30 through the internal cavity 34a of the pin 34. The electrical cables internal to the articulated arm 20 are housed in the respective internal cavities 30a, 34a, 50a of the first body 30, of the pin 34 and of the second body 50.

The first 60 and the second limitation mechanism 70 described enable a rotation of the first proximal articulation 22 greater than 360° and less than 718°. The exact extension of the rotation is equal to the sum of the angular extension of the groove 54 about the main axis of rotation R and of the angular extension of the second portion 43b of the radially internal surface 43.

The operating lamp 1 can be integrated into an operating unit 200, illustrated in Figure 8.

The operating unit 200 comprises a reclining chair 210, an articulated arm 20 of the type described above connected to the reclining chair 210 at the first distal articulation and an operating lamp 1 connected to the regulation body 21 of the articulated arm 20.

## Claims

1. Operating lamp (1) comprising:
- a light head (10) having a main light axis (L);
- a first body (30) connected to said light head (10);
- a second body (50) rotatably connected to said first body (30) about a main axis of rotation (R);
- an intermediate body (40) arranged between said first and second body (30, 50) and rotatable about said main axis of rotation (R) with respect to said first body (30) and with respect to said second body (50);
- a first limitation mechanism (60) comprising:
a first element (61) arranged on the intermediate body (40) and a second element (62) arranged on the first body (30), or
a first element (61) arranged on the first body (30) and a second element (62) arranged on the intermediate body (40); and
- a second limitation mechanism (70) comprising:
a first element (71) arranged on the intermediate body (40) and a second element (72) arranged on the second body (50), or
a first element (71) arranged on the second body (50) and a second element (72) arranged on the intermediate body (40); wherein the first element (61) and the second element (62) of the first limitation mechanism (60) cooperate to limit a rotation of the first body (30) with respect to the intermediate body (40) about the main axis of rotation (R) to a value less than 359°; and
wherein the first element (71) and the second element (72) of the second limitation mechanism (70) cooperate to limit a rotation of the intermediate body (40) with respect to the second body (50) about the main axis of rotation (R) to a value less than 359°.

2. Operating lamp (1) according to claim 1, wherein the first element (61) of the first limitation mechanism (60) comprises a first end stop (47) configured to cooperate with the second element (62) of the first limitation mechanism (60) for stopping the rotation of the first body (30) with respect to the intermediate body (40) along a first angular direction; and wherein the first element (61) of the first limitation mechanism (60) comprises a second end stop (48) configured to cooperate with the second element (62) of the first limitation mechanism (60) for stopping the rotation of the first body (30) with respect to the intermediate body (40) along a second angular direction opposite the first angular direction.

3. Operating lamp (1) according to claim 1 or 2, wherein the second element (62) of the first limitation mechanism (60) comprises a slider (33) configured to cooperate with the first element (61) of the first limitation mechanism (60) for stopping the rotation of the first body (30) with respect to the intermediate body (40).

4. Operating lamp (1) according to claims 2 and 3, wherein the first element (61) of the first limitation mechanism (60) comprises a guide track (46) between the respective first end stop (47) and a second end stop (48), the slider (33) of the first limitation mechanism (60) being configured to slide in said guide track (46) during a rotation of the first body (30) with respect to the intermediate body (40).

5. Operating lamp (1) according to one or more of the preceding claims, wherein the first element (71) of the second limitation mechanism (70) comprises a first end stop (57) configured to cooperate with the second element (72) of the second limitation mechanism (70) for stopping the rotation of the intermediate body (40) with respect to the second body (50) along a first angular direction; and wherein the first element (71) of the second limitation mechanism (70) comprises a second end stop (58) configured to cooperate with the second element (72) of the second limitation mechanism (70) for stopping the rotation of the intermediate body (40) with respect to the second body (50) along a second angular direction.

6. Operating lamp (1) according to one or more of the preceding claims, wherein the second element (72) of the second limitation mechanism (70) comprises a slider (49) configured to cooperate with the first element (71) of the second limitation mechanism (70) for stopping the rotation of the intermediate body (40) with respect to the second body (50).

7. Operating lamp (1) according to claims 5 and 6, wherein the first element (71) of the second limitation mechanism (70) comprises a guide track (56) between the respective first end stop (57) and a second end stop (58), the slider (49) of the second limitation mechanism (70) being configured to slide in said guide track (56) during a rotation of the intermediate body (40) with respect to the second body (50).

8. Operating lamp (1) according to claims 4 and 7, wherein said first body (30) and second body (50) are rotatably connected by means of a pin (34) extending along said main axis of rotation (R) and wherein the intermediate body (40) is crossed by said pin (34).

9. Operating lamp (1) according to claim 8, wherein the intermediate body (40) comprises:
- a first portion (43a) of a radially internal surface (43) conformed to slide on said pin (34) for rotatably connecting said intermediate body (40) to said pin (34) having a radial distance (D2) from the main axis of rotation (R); and
- a second portion (43b) of the radially internal surface (43) circumferentially extending from the first to the second end stop (47, 48) of one from between the first (60) and the second limitation mechanism (70) and having a radial distance (D3) from the main axis of rotation (R) greater than the radial distance (D2) from the main axis of rotation (R) of said first portion (43a);
wherein the guide track (46, 56) of said one from between the first and the second limitation mechanism (60, 70) is delimited by the second portion (43b) of the radially internal surface (43) and by the first (47, 57) and by the second end stop (48, 58) of said one from between the first (60) and the second limitation mechanism (70).

10. Operating lamp (1) according to claim 9, wherein the second portion (43b) of the radially internal surface (43) directly faces the pin (34) and the guide track (46) of said one from between the first (60) and the second limitation mechanism (70) is delimited internally by the pin (34).

11. Operating lamp (1) according to claim 10, wherein the first portion (43a) of the radially internal surface (43) circumferentially extends from the first to the second end stop (47, 48) and is complementary to the second portion (43b) of the radially internal surface (43) of the main axis of rotation in the formation of a 360° angle.

12. Operating lamp (1) according to claims 4 and 7, wherein the first and the second body (30, 50) comprise respective abutment portions (31, 51) arranged in contact abutment against the intermediate body (40), and wherein
the guide track (56) of the second limitation mechanism (70) is defined by a groove (54) obtained on the abutment portion (51) of the second body (50) and circumferentially extending around the main axis of rotation (R) from the first end stop (57) to the second end stop (58) of the second limitation mechanism (70) and the slider (49) of the second limitation mechanism (70) is connected to the intermediate body (40) and extends axially into the groove (54); or
the guide track (46) of the first limitation mechanism (60) is defined by a groove obtained on the abutment portion (31) of the first body (30) and circumferentially extending around the main axis of rotation (R) from the first end stop (47) to the second end stop (48) of the first limitation mechanism (60) and the slider (33) of the first limitation mechanism (60) is connected to the intermediate body (40) and extends axially into the groove.

13. Operating lamp (1) according to one or more of the preceding claims, wherein the intermediate body (40) comprises a washer (41) having a first flat surface (41a) arranged in contact support against said first body (30) and a second flat surface (41b) opposite the first flat surface (41a) and arranged in contact support against said second body (50).

14. Operating lamp assembly (100) comprising:
- an articulated arm (20);
- an operating lamp (1) according to one or more of the preceding claims coupled to the articulated arm (20).

15. Operating unit (200) comprising:
- a chair (210);
- an articulated arm (20) coupled to the chair (210);
- an operating lamp (1) according to one or more of claims 1 to 13, coupled to the articulated arm (20).
